# EUROPEAN PATENT SPECIFICATION

(11) **EP 0 372 843 B1**
(45) Date of publication and mention of the grant of the patent: **06.09.1995**
(21) Application number: 89312536.9
(22) Date of filing: 01.12.1989
(51) Int. Cl.: G01N 22/04, G01N 33/28

(54) **Petroleum stream microwave watercut monitor**
Anzeigegerät für den Wassergehalt eines Petroleumstromes
Dispositif d'indication du contenu de l'eau d'un courant de pétrole

(30) Priority: 05.12.1988 US 280079; 23.02.1989 US 314338
(43) Date of publication of application: 13.06.1990
(73) Proprietor: TEXACO DEVELOPMENT CORPORATION, White Plains, New York 10650 (US)
(72) Inventor: Helms, David Albert, Houston, TX 77096 (US); Marrelli, John David, Houston, TX 77009 (US); Hatton, Gregory John, Houston, TX 77035 (US); Durrett, Michael Gregory, Houston, TX 77080 (US)
(74) Representative: Wood, Anthony Charles

(56) References cited:
- EP-A- 0 268 399
- US-A- 3 818 333
- US-A- 4 499 418
- IEEE TRANSACTIONS ON INDUSTRIAL ELECTRONICS AND CONTROL INSTRUMENTATION, vol. IECI-23, no. 4, November 1976, pages 364-370; A. KRASZEWSKI et al.: "An improved microwave method of moisture content measurement and control"
- IEEE TRANSACTIONS ON INSTRUMENTS AND MEASUREMENT, vol. IM-35, no. 4, part 2,December 1986, pages 630-637; AGGARWAL and JOHNSTON: "Oil and water contentmeasurement of sandstone cores using microwave measurement techniques"
- Journal of Microwave Power, Vol. 15, No. 4, 1980, pages 209 - 220

## Description

The present invention relates to petroleum stream monitors in general and, more particularly, to petroleum stream water cut monitors.

US-A-4499418 describes a petroleum stream microwave water cut monitor comprising:
a test cell for having a petroleum stream flow through it while permitting microwave energy to enter the test cell;
a source for providing microwave energy;
an antenna for irradiating the stream flowing through the test cell with microwave energy and for receiving reflected microwave energy back from the stream in the test cell;
a circulator connected to the source and to the antenna for providing the microwave energy from the source to the antenna and for providing reflected microwave energy from the antenna as test microwave energy; and
phase indicator means connected to the source and to the circulator for providing a phase difference signal representative of the phase difference between the microwave energy provided by the source and the test microwave energy.

The present invention is characterized by:
a detector connected to the circulator for detecting the intensity of the test microwave energy and providing an intensity signal representative thereof; and
an indicator connected to the detector and the phase means for providing an indication of the water cut of the petroleum stream in accordance with said intensity signal and said phase difference signal.

Embodiments of the invention will now be described by way of example and with reference to the accompanying drawings, in which:-
Figure 1 is a combination simplified block diagram and a schematic of a watercut monitor.
Figure 2 is a side elevation of an isolator of the apparatus of Figure 1;
Figure 3A is a side elevation of one of the wave guides forming the isolator of Figure 2;
Figure 3B is a front elevation of the wave guide of Figure 3A; and
Figure 4 is a partial simplified block diagram of a microwave water cut monitor according to the present invention.

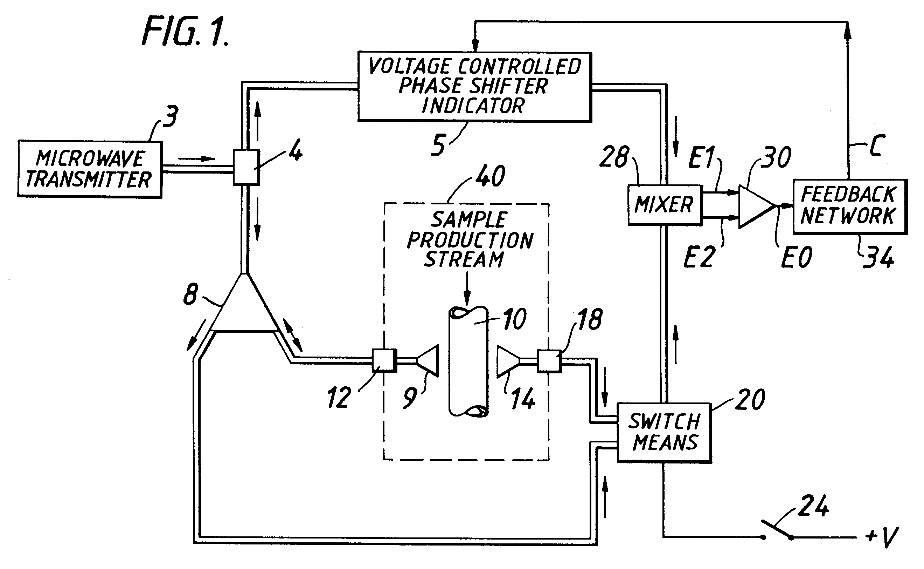

The water cut monitor shown in Figure 1 includes a microwave source 3 providing electromagnetic energy, hereinafter referred to as microwave energy, at a microwave frequency. Source 3 is low powered and may use a microwave Gunn source. Source 3 provides microwave energy to directional coupler 4. Directional coupler 4 provides microwave energy to a conventional type voltage controlled phase shifter 5 and to a circulator 8. All conductance or carrying of microwave energy is accomplished by using conventional type waveguides and coaxial cable.

Circulator 8 provides microwave energy to an antenna 9 which has, in this embodiment, an isolator 12 interconnecting antenna 9 to circulator 8. Antenna 9 transmits or radiates the microwave energy through a sample stream of a fluid mixture passing through a test cell 10. Test cell 10 may be a portion of a pipeline with "windows" made of material which permits passage of the microwave energy or it may be a portion of the pipeline made of the "window" material. The transmitted microwave energy passes through the fluid mixture and is received by an antenna 14 which provides the received microwave energy to a switch means 20.

The fluid mixture also reflects some of the microwave energy back to antenna 9 which passes back through antenna 9 to circulator 8. Circulator 8 blocks the reflected microwave energy from feeding back to source 3 and provides the reflected microwave energy to switch means 20. Reflected microwave energy becomes more important as the distance between antennas 9 and 14 increases. This is especially true where a large pipeline carrying the fluid mixture is being monitored.

A positive direct current voltage +V is provided to a switch means 24 which is connected to switch means 20. With switch means 24 open, switch means 20 provides microwave energy from antenna 14 as test microwave energy. When switch 24 is closed, the reflected microwave energy from circulator 8 is provided by switch means 20 as the test microwave energy.

The microwave energy from voltage control phase shifter 5, hereinafter called the reference microwave energy, and the test microwave energy from switch 20, are provided to a mixer 28 which mixes them to provide two electrical signals E1, E2, representative of the phases of the reference microwave energy and the test microwave energy.

A differential amplifier 30 provides an output signal E0 in accordance with the difference between signals E1 and E2. Signal E0 is a function of the phase difference between the reference microwave energy and the test microwave energy. Signal E0 may be applied to an indicator in which the amplitude of E0 will be representative of the phase difference or as shown in the present example may be provided to a feedback network 34. Feedback network 34 provides a control voltage C to voltage control phase shifter 5 controlling the phase of the reference microwave energy. Signal E0, and hence the control voltage C, decreases in amplitude until there is substantially 90° phase difference between the reference microwave energy and the test microwave energy. Voltage control phase shifter 5 indicates the amount of phase shift required to eliminate the phase difference.

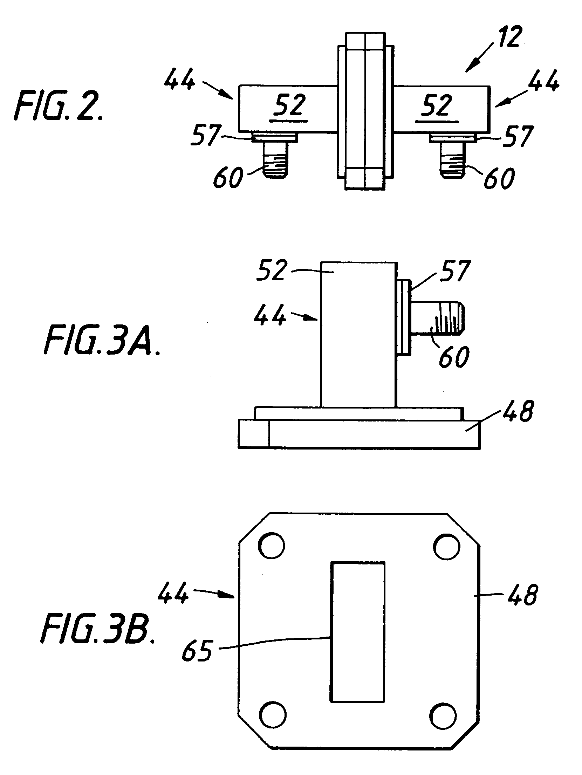

With reference to Figure 2, there is shown isolator 12. Isolator 12 is used to protect an area adjacent test cell 10 from potential explosions. This unsafe area, which may be of any size, is shown in Figure 1 by dashed line 40.

The possibility of an explosion arises since many of the energy controlling components of the watercut monitor are made of metal for optimal microwave transmission. As such, all energy generated in the watercut circuits could be conducted to antennas 9 and 14. Under normal operating conditions, energy is limited to safe levels not capable of causing an explosion. In the event of accident or misuse of the watercut monitor much higher energy and energy of non-microwave frequencies could be applied to antennas 9 and 14.

Isolators 12 and 18 isolate antennas 9 and 14 from the buildup voltages that may occur on any of the other elements and thus protect them from arcing and causing an explosion.

Isolator 12 is made up of two identical wave guides 44 which is shown in Figures 3A and 3B. Wave guides 44 include mounting flanges 48, a guide element 52, insulators 57 and a metal jack 60. Metal jack 60 is isolated from the wave guide 52 so that any voltage buildup on jack 60 does not appear on guide element 52. As can be seen in Figure 3B, the RF energy aperture is identified by the numeral 65. It should be noted that although isolator 12 is shown with both jacks 60 facing downward it is obvious to one skilled in the art that one of the wave guides 44 may be reorientated 180° without loss of cross-sectional area of aperture 65, if it is so desired to do so.

The present embodiment has been described as being used with a water cut monitor of the type described and disclosed in U.S.P. 4,499,418, but it may also be used in either situation where a water cut monitor uses microwave energy singularly either as a direct passthrough type of measurement or in another configuration as a reflected microwave energy configuration.

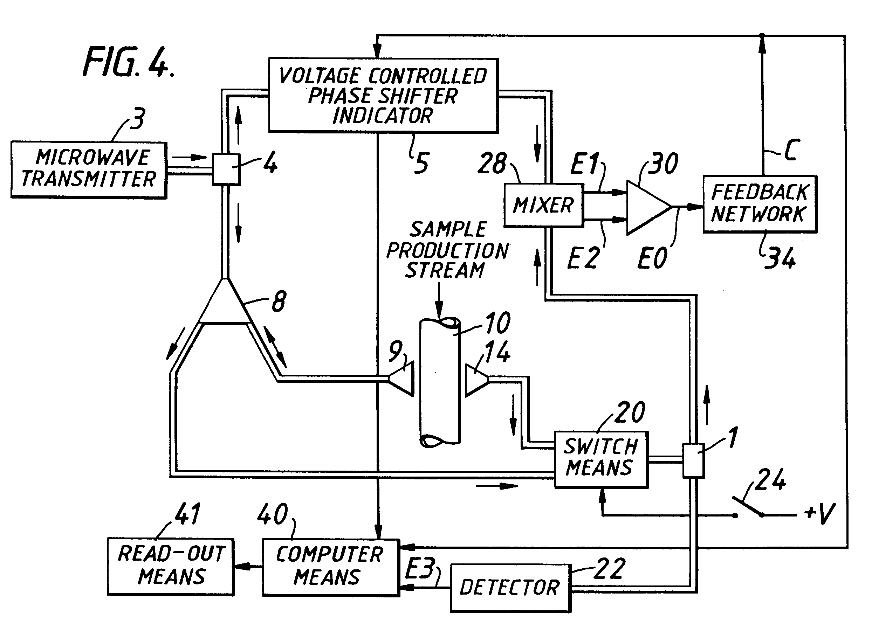

The embodiment of the invention shown in Figure 4 is a modification of the apparatus shown in Figure 1. The same parts have been given the same reference numerals in Figures 1 and 4. The description of this embodiment will be restricted to the modified portions of the apparatus of the Figure 4 embodiment.

In the Figure 4 embodiment the switch means 20 provides test microwave energy to a directional coupler 19. Directional coupler 19 provides the test microwave energy to a detector 22 as well as to mixer 28. Detector 22 provides a signal E3 corresponding to the intensity of the microwave energy received by antenna 14. Feedback network 34 provides signal C to voltage control phase shifter 5, controlling the phase of the reference microwave energy, and also to a mini-computer means 40. Signals E3 and C are provided to mini-computer means 40 which contains within it memory means having data related to phase and amplitude for various percentages of water cuts that could be encountered in the production stream. Phase Shifter 5 also provides an enabling signal to computer means 40 allowing computer means 40 to utilize signals C and E3 to select the proper water cut value computer means 40 provides signals, corresponding to the selected water cut value, to readout means 41 which may be either digital display means or record means or a combination of the two.

## Claims

1. A petroleum stream microwave water cut monitor comprising:
a test cell (10) for having a petroleum stream flow through it while permitting microwave energy to enter the test cell;
a source (3) for providing microwave energy;
an antenna (9) for irradiating the stream flowing through the test cell with microwave energy and for receiving reflected microwave energy back from the stream in the test cell;
a circulator (8) connected to the source and to the antenna for providing the microwave energy from the source to the antenna and for providing reflected microwave energy from the antenna as test microwave energy; and
phase indicator means (5,28,30,34) connected to the source and to the circulator for providing a phase difference signal (C) representative of the phase difference between the microwave energy provided by the source and the test microwave energy;
characterized by:
a detector (22) connected to the circulator for detecting the intensity of the test microwave energy and providing an intensity signal (E3) representative thereof; and
an indicator (40,41) connected to the detector and the phase means for providing an indication of the water cut of the petroleum stream in accordance with said intensity signal (E3) and said phase difference signal (C).

2. A monitor according to claim 1 characterized in that the phase means (5,28,30,34) comprises:
a voltage controlled phase shifter (5) connected to the source (3) to receive microwave energy provided by said source, to shift the phase of the provided microwave energy in accordance with a phase shift signal to provide reference microwave energy, and to provide an enabling signal when the phase shifting is completed; and
phase shift signal means (28,30,34) connected to receive said reference microwave energy and said test microwave energy for providing said phase shift signal to the phase shifter (5) until there is substantially a 90° phase difference between the reference microwave energy and the test microwave energy at which time the phase shifting is completed and said enabling signal is provided to said indicator (40,41) to enable said indicator to provide said indication.

3. A monitor according to claim 2 characterized in that the phase shift signal means (28,30,34) includes:
a mixer (28) connected to the phase shifter (5) and to the circulator (8) for mixing the reference microwave energy from the phase shifter with the test microwave energy from the circulator to provide two signals (E1,E2) representative respectively of the phases of the reference microwave energy and the test microwave energy;
a differential amplifier (30) connected to the mixer for providing an output signal (EO) in accordance with the difference between the two signals from the mixer; and
a feedback network (34) connected to the phase shifter and to the differential amplifier to provide said phase shift signal in accordance with the output signal.

4. A monitor according to any one of claims 1 to 3 characterized by an isolator (12) connected between the circulator (8) and the antenna (9) for passing microwave energy to and from the antenna while isolating the antenna from extraneous energies that may arise in the water cut monitor so as to prevent an accidental explosion due to those extraneous energies.

5. A monitor according to any one of claims 1 to 3 characterized by:
a second antenna (14) for receiving microwave energy that has passed through the stream flowing in the test cell to provide received microwave energy; and
a switch (20) operable to provide said received microwave energy as said test microwave energy in place of the microwave energy from the circulator.

6. A monitor according to claim 5 characterized by:
a first isolator (12) connected between the circulator (8) and the first-mentioned antenna (9) for passing microwave energy to and from the first-mentioned antenna while isolating the first-mentioned antenna from extraneous energies that may arise in the water cut monitor so as to prevent an accidental explosion due to those extraneous energies; and
a second isolator (18) connected between the second antenna (14) and the switch (20) for passing received microwave energy from the second antenna to the switch while isolating the second antenna from extraneous energies that may arise in the water cut monitor so as to prevent an accidental explosion due to those extraneous energies.

## Patentansprüche

1. Mikrowellen-Petroleumstromwassergehaltmeßgerät mit:
einer Prüfzelle (10) zum Durchfließen eines Petroleumstroms bei gleichzeitigem Eintritt von Mikrowellenenergie in die Prüfzelle;
einer Quelle (3) zur Bereitstellung von Mikrowellenenergie;
einer Antenne (9) zum Bestrahlen des die Prüfzelle durchfließenden Stromes mit Mikrowellenenergie und zum Zurückempfangen von reflektierter Mikrowellenenergie vom Strom in der Prüfzelle;
einem mit der Quelle und mit der Antenne verbundenen Zirkulator (8) zur Bereitstellung der Mikrowellenenergie von der Quelle zur Antenne und zur Bereitstellung von reflektierter Mikrowellenenergie von der Antenne als Prüfmikrowellenenergie; und
mit der Quelle und mit dem Zirkulator verbundenen Phasenanzeigermitteln (5,28,30,34) zur Bereitstellung eines die Phasendifferenz zwischen der von der Quelle bereitgestellten Mikrowellenenergie und der Prüfmikrowellenenergie darstellenden Phasendifferenzsignals (C), gekennzeichnet durch:
einen mit dem Zirkulator verbundenen Detektor (22) zum Erfassen der Intensität der Prüfmikrowellenenergie und Bereitstellen eines diese darstellenden Intensitätssignals (E3);
einen mit dem Detektor und dem Phasenmittel verbundenen Anzeiger (40,41) zur Bereitstellung einer Anzeige des Wassergehalts des Petroleumstroms entsprechend dem besagten Intensitätssignal (E3) und besagten Phasendifferenzsignal (C).

2. Meßgerät nach Anspruch 1, dadurch gekennzeichnet, daß die Phasenmittel (5,28,30,34) folgendes umfassen:
einen mit der Quelle (3) verbundenen spannungsgesteuerten Phasenschieber (5) zum Empfangen der von der besagten Quelle bereitgestellten Mikrowellenenergie, zum Verschieben der Phase der bereitgestellten Mikrowellenenergie entsprechend einem Phasenverschiebungssignal zur Bereitstellung von Bezugsmikrowellenenergie und zur Bereitstellung eines Freigabesignals, wenn die Phasenverschiebung vollendet ist; und
zum Empfangen der besagten Bezugsmikrowellenenergie und der besagten Prüfmikrowellenenergie verbundene Phasenverschiebungssignalmittel (28,30,34) zur Bereitstellung des besagten Phasenverschiebungssignals zum Phasenschieber (5), bis im wesentlichen ein Phasenunterschied von 90° zwischen der Bezugsmikrowellenenergie und der Prüfmikrowellenenergie besteht, wonach die Phasenverschiebung vollendet ist und das besagte Freigabesignal dem besagten Anzeiger (40,41) zur Freigabe des besagten Anzeigers zur Bereitstellung der besagten Anzeige zugeführt wird.

3. Meßgerät nach Anspruch 2, dadurch gekennzeichnet, daß die Phasenverschiebungssignalmittel (28,30,34) folgendes enthalten:
einen mit dem Phasenschieber (5) und dem Zirkulator (8) verbundenen Mischer (28) zum Vermischen der Bezugsmikrowellenenergie vom Phasenschieber mit der Prüfmikrowellenenergie vom Zirkulator zur Bereitstellung von zwei die Phasen der Bezugsmikrowellenenergie bzw. der Prüfmikrowellenenergie darstellenden Signalen (E1,E2);
einen mit dem Mischer verbundenen Differenzverstärker (30) zur Bereitstellung eines Ausgangssignals (EO) entsprechend der Differenz zwischen den zwei Signalen vom Mischer; und
ein mit dem Phasenschieber und mit dem Differenzverstärker verbundenes Rückkoppelnetz (34) zur Bereitstellung des besagten Phasenverschiebungssignals entsprechend dem Ausgangssignal.

4. Meßgerät nach einem beliebigen der Ansprüche 1 bis 3, gekennzeichnet durch einen zwischen den Zirkulator (8) und die Antenne (9) geschalteten Einwegleiter (12) zum Weiterleiten von Mikrowellenenergie zu und von der Antenne und gleichzeitigem Abtrennen der Antenne von Außenenergien, die im Wassergehaltmeßgerät entstehen können, um eine zufällige Explosion aufgrund dieser Außenenergien zu verhindern.

5. Meßgerät nach einem beliebigen der Ansprüche 1 bis 3, gekennzeichnet durch:
eine zweite Antenne (14) zum Empfangen von Mikrowellenenergie, die den in der Prüfzelle fließenden Strom durchlaufen hat, um Empfangsmikrowellenenergie bereitzustellen; und
einen Schalter (20) zur Bereitstellung der besagten Empfangsmikrowellenenergie als besagte Prüfmikrowellenenergie anstatt der Mikrowellenenergie vom Zirkulator.

6. Meßgerät nach Anspruch 5, gekennzeichnet durch:
einen ersten zwischen den Zirkulator (8) und die ersterwähnte Antenne (9) geschalteten Einwegleiter (12) zum Weiterleiten von Mikrowellenenergie zu und von der ersterwähnten Antenne und gleichzeitigen Abtrennen der ersterwähnten Antenne von Außenenergien, die im Wassergehaltmeßgerät entstehen können, um eine zufällige Explosion aufgrund dieser Außenenergien zu verhindern; und
einen zweiten zwischen die zweite Antenne (14) und den Schalter (20) geschalteten Einwegleiter (18) zum Weiterleiten von Empfangsmikrowellenenergie von der zweiten Antenne zum Schalter und gleichzeitigen Abtrennen der zweiten Antenne von Außenenergien, die im Wassergehaltmeßgerät entstehen können, um eine zufällige Explosion aufgrund dieser Außenenergien zu verhindern.

## Revendications

1. Dispositif micro-ondes d'indication de la teneur en eau d'un courant de pétrole comprenant:
une cellule d'essai (10) pour faire s'écouler un courant de pétrole en son travers tout en permettant à de l'énergie micro-ondes de pénétrer dans la cellule d'essai;
une source (3) pour fournir l'énergie micro-ondes;
une antenne (9) pour irradier le courant s'écoulant à travers la cellule d'essai avec de l'énergie micro-ondes et pour recevoir l'énergie micro-ondes réfléchie depuis le courant dans la cellule d'essai;
un organe de circulation (8) raccordé à la source et à l'antenne pour fournir l'énergie micro-ondes de la source à l'antenne et pour fournir de l'énergie micro-ondes réfléchie depuis l'antenne en tant qu'énergie micro-ondes d'essai; et
un moyen d'indication de phase (5, 28, 30, 34) raccordé à la source et à l'organe de circulation pour fournir un signal (C) de déphasage représentatif du déphasage entre l'énergie micro-ondes fournie par la source et l'énergie micro-ondes d'essai;
caractérisé par:
un détecteur (22) raccordé à l'organe de circulation pour détecter l'intensité de l'énergie micro-ondes d'essai et fournir un signal d'intensité (E3) représentatif de cette dernière; et
un indicateur (40, 41) raccordé au détecteur et au moyen d'indication de phase pour fournir une indication de la teneur en eau du courant de pétrole en conformité avec ledit signal d'intensité (E3) et ledit signal de déphasage (C).

2. Dispositif d'indication selon la revendication 1, caractérisé en ce que le moyen d'indication de phase (5, 28, 30, 34) comprend:
un organe de décalage de phase (5) commandé par tension raccordé à la source (3) pour recevoir l'énergie micro-ondes fournie par ladite source, pour décaler la phase de l'énergie micro-ondes fournie en conformité avec un signal de décalage de phase pour fournir une énergie micro-ondes de référence, et pour fournir un signal d'activation quand le décalage de phase est complété; et
un moyen de signal de décalage de phase (28, 30, 34) raccordé pour recevoir ladite énergie micro-ondes de référence et ladite énergie micro-ondes d'essai pour fournir ledit signal de décalage de phase à l'organe de décalage de phase (5) jusqu'à ce qu'il y ait substantiellement un déphasage de 90° entre l'énergie micro-ondes de référence et l'énergie micro-ondes d'essai auquel moment le décalage de phase est complété et ledit signal d'activation est fourni audit indicateur (40, 41) pour permettre audit indicateur de fournir ladite indication.

3. Dispositif d'indication selon la revendication 2, caractérisé en ce que le moyen de signal de décalage de phase (28, 30, 34) inclut:
un mélangeur (28) raccordé à l'organe de décalage de phase (5) et à l'organe de circulation (8) pour mélanger l'énergie micro-ondes de référence issue de l'organe de décalage de phase avec l'énergie micro-ondes d'essai issue de l'organe de circulation pour fournir deux signaux (E1, E2) représentatifs respectivement des phases de l'énergie micro-ondes de référence et de l'énergie micro-ondes d'essai;
un amplificateur différentiel (30) raccordé au mélangeur pour fournir un signal de sortie (EO) en conformité avec la différence entre les deux signaux issus du mélangeur; et
un réseau de rétroaction (34) raccordé à l'organe de décalage de phase et à l'amplificateur différentiel pour fournir ledit signal de décalage de phase en conformité avec le signal de sortie.

4. Dispositif d'indication selon l'une quelconque des revendications 1 à 3, caractérisé par un isolateur (12) raccordé entre l'organe de circulation (8) et l'antenne (9) pour faire passer l'énergie micro-ondes jusqu'à et depuis l'antenne tout en isolant l'antenne des énergies étrangères qui peuvent survenir dans le dispositif d'indication de la teneur en eau de manière à empêcher une explosion accidentelle due à ces énergies étrangères.

5. Dispositif d'indication selon l'une quelconque des revendications 1 à 3, caractérisé par:
une deuxième antenne (14) pour recevoir l'énergie micro-ondes qui a passé à travers le courant s'écoulant dans la cellule d'essai pour fournir l'énergie micro-ondes reçue; et
un moyen de commutation (20) pouvant fonctionner pour fournir ladite énergie micro-ondes reçue en tant que ladite énergie micro-ondes d'essai à la place de l'énergie micro-ondes issue de l'organe de circulation.

6. Dispositif d'indication selon la revendication 5, caractérisé par:
un premier isolateur (12) raccordé entre l'organe de circulation (8) et l'antenne (9) mentionnée en premier lieu pour faire passer l'énergie micro-ondes jusqu'à et depuis l'antenne mentionnée en premier lieu tout en isolant l'antenne mentionnée en premier lieu des énergies étrangères qui peuvent survenir dans le dispositif d'indication de la teneur en l'eau de manière à empêcher une explosion accidentelle due à ces énergies étrangères; et
un deuxième isolateur (18) raccordé entre la deuxième antenne (14) et le moyen de commutation (20) pour faire passer l'énergie micro-ondes reçue depuis la deuxième antenne jusqu'au moyen de commutation tout en isolant la deuxième antenne des énergies étrangères qui peuvent survenir dans le dispositif d'indication de la teneur en eau de manière à empêcher une explosion accidentelle due à ces énergies étrangères.
